# FASCICULE DE BREVET EUROPEEN

(11) **EP 1 963 355 B1**
(45) Date de publication et mention de la délivrance du brevet: **30.09.2009**
(21) Numéro de dépôt: 06841943.1
(22) Date de dépôt: 15.12.2006
(51) Int. Cl.: C07J 41/00, A61K 31/575, A61P 25/28, A61P 25/00

(54) **NOUVEAUX DERIVES DE L'OXIME DE CHOLEST-4-EN-3-ONE, COMPOSITIONS PHARMACEUTIQUES LES RENFERMANT, ET PROCEDE DE PREPARATION**
NEUE CHOLEST-4-EN-3-ONOXIMDERIVATE, PHARMAZEUTISCHE ZUSAMMENSETZUNGEN, DIE DIESE ENTHALTEN, UND HERSTELLUNGSVERFAHREN
NEW CHOLEST-4-EN-3-ONE OXIME DERIVATIVES, PHARMACEUTICAL COMPOSITIONS COMPRISING THEM, AND PREPARATION PROCESS

(30) Priorité: 20.12.2005 FR 0512947
(43) Date de publication de la demande: 03.09.2008
(73) Titulaire: Trophos, 13288 Marseille Cedex 9 (FR)
(72) Inventeur: DROUOT, Cyrille, F-83300 Draguignan (FR); MAUX, Delphine, F-06130 Grasse (FR)
(74) Mandataire: Galup, Cédric Olivier Nicolas
(86) Numéro de dépôt international: PCT/FR2006/002740
(87) Numéro de publication internationale: WO 2007/080270

(56) Documents cités:
- WO-A2-20/04082581
- ES-A1- 2 190 348
- FR-A1- 2 852 246
- FR-A1- 2 860 159
- SINGH, HARKISHAN; PAUL, DHARAM; BHARDWAJ, TILAK RAJ; BHUTANI, KAMLESH K.; RAM, JAISHI: "Steroids and related studies. XL. Thin-layer chromatography of some steroidal ketones, oximes and lactams" JOURNAL OF CHROMATOGRAPHY, vol. 137, no. 1, 1977, pages 202-205, XP002402558
- OKA, KITARO; HARA, SHOJI: "Regiospecific Beckmann rearrangement of 3-oxo-4-ene steroid oximes" JOURNAL OF ORGANIC CHEMISTRY, vol. 43, no. 19, 1978, pages 3790-3791, XP002402559
- HUSAIN, MUBARAK; KHAN, NASEEM HASAN: "Reaction of chlorotrimethylsilane with some cholestane derivatives" SYNTHETIC COMMUNICATIONS, vol. 11, no. 3, 1981, pages 185-207, XP008069907
- PINHEY, JOHN T.; RIZZARDO, EZIO; SMITH, GEOFFREY C.: "The photochemistry of 3.beta.-acetoxy-6-nitrocholest-5-ene and 6-nitrocholest-5-ene. The mechanism of photodeconjugated of .alpha.,.beta.-unsaturated nitro compounds" AUST. J. CHEM., vol. 31, 1978, pages 97-112, XP008069948
- SINGH, HARKISHAN; BHUTANI, KAMLESH K.: "Steroids and related studies. Part XLII. Tetrazoles from cholest-4-ene-3,6-dione, 3-oximinocholest-4-ene-3,6-dione and (25R)-spirost-4-ene-3,6-dione" INDIAN JOURNAL OF CHEMISTRY, SECTION B:, vol. 16b, 1978, pages 95-97, XP008069942
- DR. W. WALTER, DR. DR.H.C. W. FRANCKE: "Lehrbuch der Organischen Chemie" 1998, S. HIRZEL , LEIPZIG , XP002438804 page 736 page 741

## Description

La présente invention concerne l'application à titre de médicaments de dérivés de l'oxime de cholest-4-èn-3-one, notamment comme médicaments cytoprotecteurs, particulièrement comme médicaments neuroprotecteurs ou cardioprotecteurs. Lesdits médicaments sont particulièrement adaptés aux pathologies et aux traumatismes liés à la dégénérescence ou à la mort cellulaire, particulièrement celles des motoneurones et/ou des cellules cardiaques. L'invention concerne également les compositions pharmaceutiques renfermant lesdits composés, de nouveaux dérivés et leur procédé de préparation.

Les processus dégénératifs cellulaires sont caractérisés par le dysfonctionnement des cellules entraînant souvent des activités cellulaires indésirables et la mort cellulaire.

Les cellules ont développé des mécanismes d'adaptation, en réponse au stress, qui allongent leur durée de vie ou retardent ou empêchent la mort cellulaire (mécanismes cytoprotecteurs).

Cependant, ces mécanismes cytoprotecteurs sont parfois insuffisants, inadéquats, ou induits trop tard pour être efficaces et les cellules meurent. Il peut donc s'avérer intéressant de disposer de nouveaux médicaments, cytoprotecteurs, qui favoriseraient la cytoprotection. C'est un des buts de la présente invention.

Parmi les mécanismes principaux de mort cellulaire, on distingue essentiellement la nécrose, l'apoptose et la nécroptose.

La nécrose est une mort cellulaire dite "accidentelle" qui survient lors d'un dommage tissulaire. C'est la membrane plasmique de la cellule qui est la plus touchée, entraînant une modification de l'homéostasie de la cellule. Les cellules vont se gorger d'eau au point que cela va entraîner la lyse de leur membrane plasmique. Cette lyse cellulaire conduit au largage dans le milieu environnant du contenu cytoplasmique. La nécrose est à l'origine du processus inflammatoire.

La nécrose peut toucher un ensemble de cellules ou un tissu alors que les autres parties de voisinage restent vivantes. La transformation qui en résulte est une mortification des cellules ou des tissus.

Autrement dit, la nécrose se définit par des modifications morphologiques survenant lorsqu'une cellule arrive en fin de vie à la suite d'événements tels qu'un traumatisme important comme un arrêt ou une diminution de la circulation sanguine au niveau d'un organe, l'hyperthermie (élévation importante de la température), une intoxication par un produit chimique, un choc physique, etc...

Une des nécroses les plus connues est celle du myocarde lors de l'infarctus (arrêt d'apport circulatoire au niveau du muscle cardiaque) due à une oblitération (obstruction) d'une artère coronaire.

L'apoptose fait partie intégrante de la physiologie normale d'un organisme. C'est une forme physiologique de mort cellulaire hautement régulée et elle est nécessaire à la survie des organismes multicellulaires. L'apoptose est un processus qui joue un rôle primordial au cours de l'embryogenèse.

Les cellules en apoptose ou apoptotiques vont s'isoler des autres cellules. L'apoptose implique habituellement des cellules individuelles dans un tissu et ne provoque pas l'inflammation. L'un des points morphologiques caractéristiques de l'apoptose est l'importante condensation à la fois du noyau et du cytoplasme ce qui induit une diminution significative du volume cellulaire. Le noyau se fragmente ensuite, chaque fragment est entouré d'une double enveloppe. Des corps apoptotiques (éléments cytoplasmiques et nucléaires) sont ensuite libérés et vont être absorbés par phagocytose par les cellules voisines.

L'apoptose peut être induite de différentes façons. Par exemple, une radiation, la présence d'un composé chimique ou d'une hormone sont des stimuli susceptibles d'induire une cascade d'événements apoptotiques dans la cellule. Des signaux intracellulaires comme une mitose incomplète ou un dommage à l'ADN peuvent aussi induire l'apoptose.

L'apoptose intervient aussi après l'action d'un génotoxique ou au cours d'une maladie. Certaines pathologies sont caractérisées par une apoptose anormale, entraînant la perte de certaines populations cellulaires, comme par exemple l'hépatotoxicité, les rétinopathies, la cardiotoxicité.

On distingue donc l'apoptose physiologique et l'apoptose pathologique. L'invention s'adresse essentiellement à l'apoptose pathologique.

Il existe d'autres mécanismes de mort cellulaire, comme par exemple la nécroptose, qui présente des caractéristiques de la nécrose et de l'apoptose. Une cellule mourant par nécroptose présente des caractéristiques similaires à celles d'une cellule mourant par nécrose, mais les étapes biochimiques de ce mécanisme s'assimilent plus à celles de l'apoptose. Ce mécanisme de mort cellulaire intervient par exemple dans l'ischémie.

C'est donc aussi un des buts de la présente invention que de disposer de nouveaux médicaments qui pourraient permettre de prévenir et/ou traiter la nécrose et/ou l'apoptose pathologique et/ou la nécroptose (médicaments antinécrotiques et/ou antiapoptotiques et/ou antinécroptotiques).

Les processus dégénératifs cellulaires peuvent résulter, entre autres, de situations pathologiques regroupées sous le terme de maladies ou affections dégénératives, de traumatismes, ou d'exposition à divers facteurs.

Ces traumatismes et facteurs peuvent inclure, par exemple, l'exposition aux radiations (UV, gamma), l'hypoxie ou la privation d'oxygène, la privation de nutriments, la privation de facteurs de croissance, des poisons, des toxines cellulaires, des déchets, des toxines environnementales, des radicaux libres, des oxygènes réactifs ou encore certains événements et/ou procédures médicaux comme par exemple les traumatismes chirurgicaux incluant les transplantations. On peut citer également des agents chimiques ou biologiques utilisés comme agents thérapeutiques dans le contexte de traitements médicaux comme par exemple des agents cytostatiques ou des agents anti-inflammatoires.

Parmi les situations pathologiques caractérisées par un processus dégénératif les plus importantes, on trouve :
■ les maladies des os, des articulations, du tissu conjonctif et du cartilage, telles que l'ostéoporose, l'ostéomyélite, les arthrites dont par exemple l'ostéoarthrite, l'arthrite rhumatoïde et l'arthrite psoriasique, la nécrose avasculaire, la fibrodysplasie ossifiante progressive, le rachitisme, le syndrome de Cushing ;
■ les maladies musculaires telles que la dystrophie musculaire, comme par exemple la dystrophie musculaire de Duchenne, les dystrophies myotoniques, les myopathies et les myasthénies ;
■ les maladies de la peau, telles que les dermatites, l'eczéma, le psoriasis, le vieillissement, ou encore les altérations de la cicatrisation ;
■ les maladies cardiovasculaires telles que l'ischémie cardiaque et/ou vasculaire, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, l'anoxie, l'hypoxie, les effets secondaires dus à des thérapies avec des agents anticancéreux ;
■ les maladies circulatoires telles que l'athérosclérose, les scléroses artérielles, les maladies vasculaires périphériques, les accidents vasculaires cérébraux, les anévrismes ;
■ les maladies hématologiques et vasculaires telles que : l'anémie, l'amyloïdose vasculaire, les hémorragies, la drépanocytose, le syndrome de la fragmentation des globules rouges, la neutropénie, la leucopénie, l'aplasie médullaire, la pancytopénie, la thrombocytopénie, l'hémophilie ;
■ les maladies du poumon incluant la pneumonie, l'asthme ; les maladies chroniques obstructives des poumons comme par exemple les bronchites chroniques et l'emphysème ;
■ les maladies du tractus gastro-intestinal, telles que les ulcères ;
■ les maladies du foie incluant les hépatites virales et les cirrhoses, les maladies du foie dues à des toxines ou à des médicaments ;
■ les maladies du pancréas comme par exemple les pancréatites aigues
ou chroniques ;
■ les maladies métaboliques telles que les diabètes mellitus et insipide, les thyroïdites ;
■ les maladies des reins telles que par exemple les désordres rénaux aigus ou la glomérulonéphrite ;
■ les infections virales et bactériennes telle que la septicémie ;
■ les intoxications sévères par des agents chimiques, des toxines ou des médicaments ;
■ les affections dégénératives associées au Syndrome Immuno Déficitaire Acquis (SIDA) ;
■ les désordres associés au vieillissement, tel que le syndrome du vieillissement accéléré ;
■ les maladies inflammatoires, telles que la maladie de Crohn, la polyarthrite rhumatoïde ;
■ les maladies auto-immunes telles que le lupus érythémateux ;
■ les désordres dentaires tels que ceux aboutissant à la dégradation des tissus comme par exemple les périodontites ;
■ les maladies ou désordres ophtalmiques incluant les rétinopathies diabétiques, le glaucome, les dégénérescences maculaires, la dégénérescence rétinienne, la rétinite pigmentaire, les trous ou déchirures rétiniennes, le décollement rétinien, l'ischémie rétinienne, les rétinopathies aigues associées à un traumatisme, les dégénérescences inflammatoires, les complications post chirurgicales, les rétinopathies médicamenteuses, la cataracte ;
■ les désordres des voies auditives, tels que l'otosclérose et la surdité induite par des antibiotiques ;
■ les maladies associées aux mitochondries (pathologies mitochondriales), telles que l'ataxie de Friedrich, la dystrophie musculaire congénitale avec anomalie mitochondriale structurelle, certaines myopathies (syndrome des MELAS, syndrome de MERFF, syndrome de Pearson), le syndrome de MIDD (diabètes mitochondriaux et surdité), le syndrome de Wolfram, la dystonie.

Par alleurs, les processus neurodégénératifs sont caractérisés par le dysfonctionnement et la mort des neurones entraînant la perte des fonctions neurologiques médiées par le cerveau (système nerveux central, SNC), la moelle épinière et le système nerveux périphérique (SNP). Ils peuvent résulter, entre autres, de situations pathologiques regroupées sous le terme de maladies ou affections neurodégénératives, de traumatisme, ou d'exposition à des toxines.

Les pathologies les plus importantes qui sont caractérisées par un processus neurodégénératif sont :
- les maladies chroniques neurodégénératives, héréditaires ou sporadiques, notamment la maladie d'Alzheimer, la maladie de Huntington, la maladie de Parkinson, la sclérose latérale amyotrophique, les amyotrophies spinales, particulièrement infantiles, la maladie de Creutzfeldt-Jakob, la sclérose en plaque, les scléroses latérales amyotrophiques, l'adrénoleucodystrophie, l'épilepsie, les démences, la schizophrénie, et les syndromes neurologiques associés au SIDA;
- les lésions neuronales liées au vieillissement ;
- les neuropathies périphériques héréditaires ou lésionnelles, comme les maladies de Fabry, de Charcot-Marie-Tooth, de Krabbe, les leucodystrophies, les neuropathies diabétiques et celles induites par les traitements anticancéreux ;
- les traumatismes du cerveau, des nerfs périphériques ou de la moelle épinière;
- les ischémies du cerveau ou de la moelle épinière suite à un accident cérébro-vasculaire, ou induites par un manque d'irrigation sanguine ;
- les dégénérescences, héréditaires, lésionnelles ou liées au vieillissement des neurones sensoriels de la vision, comme les dégénérescences maculaires, les rétinites pigmentaires, ou les dégénérescences du nerf optique induites par les glaucomes ;
- les dégénérescences, héréditaires, traumatiques ou liées au vieillissement des neurones sensoriels de l'ouïe entraînant une diminution ou une perte de l'audition.

Une partie des voies de signalisation affectées dans ces pathologies sont communes à un grand nombre de maladies neurodégénératives. La maladie d'Alzheimer est la démence la plus fréquente. Elle fait apparaître une atrophie du cerveau, une perte neuronale prédominante dans la corne d'Ammon et elle touche aussi les neurones cholinergiques. D'autres pathologies, comme les atrophies lobaires (maladie de Pick, la maladie de Creutzfeld-Jakob), la démence avec corps de Lewy, les démences vasculaires, la maladie de Parkinson sont associées à une mort neuronale importante à l'origine des symptômes de ces démences.

Il n'existe pas actuellement de traitement efficace pour enrayer les dégénérescences neuronales. Une approche thérapeutique pour protéger les neurones de la mort est l'apport de protéines neurotrophiques.

Ces protéines, telles que BDNF (brain-derived neurotrophic factor), CNTF (ciliary neurotrophic factor), NGF (nerve growth factor), GDNF (glia-derived neurotrophic factor) sont synthétisées au cours du développement embryonnaire ou après lésion chez l'adulte. Ces facteurs de croissance favorisent la survie, la maturation et la différentiation des cellules neuronales. De plus, ils inhibent les mécanismes apoptotiques, activent de multiples voies de survie et protègent un grand nombre de populations neuronales. Leur utilisation est proposée dans la plupart des dégénérescences neuronales.

Des composés qui activeraient l'expression de facteurs neurotrophiques ou qui mimeraient l'action de ces facteurs ont un potentiel thérapeutique pour le traitement des syndromes neurodégénératifs.

En particulier, l'apport de molécules neurotrophiques pour le traitement des dégénérescences neuronales vise trois objectifs :
- compenser une carence potentielle en facteurs neurotrophiques liée à un défaut d'apport par les cibles périphériques ou centrales des neurones et/ou un trouble du transport rétrograde de ces facteurs ;
- intervenir de façon non spécifique sur des voies biochimiques impliquées dans la cascade dégénérative;
- favoriser les phénomènes compensateurs naturels de croissance dendritique et d'arborisation des terminaisons nerveuses.
   Ces composés présenteraient donc un effet bénéfique dans un grand nombre de pathologies en particulier dans les pathologies touchant les systèmes nerveux périphérique et central.
   Par ailleurs, dans le cadre ci-dessus, les motoneurones sont des neurones notamment présents dans la moelle épinière et le tronc cérébral. Leur dégénérescence ou leur mort peut conduire à une faiblesse progressive des muscles des membres, puis à une atrophie et éventuellement à une spasticité (c'est à dire une contraction permanente) du muscle.
   Les pathologies les plus importantes qui résultent de la dégénérescence et de la mort des motoneurones spinaux et/ou bulbaires sont la sclérose latérale amyotrophique, également connue sous le nom de maladie de Charcot
ou encore maladie de Lou Gehrig, et les amyotrophies spinales, particulièrement infantiles, également connues sous les noms de maladie de Werdnig-Hoffmann ou maladie de Kugelberg-Welander.

En outre, on observe une dégénérescence des motoneurones dans les cas de traumatismes avec écrasement et/ou section de la moelle épinière ou des nerfs moteurs périphériques.

Plus généralement, on parle d'amyotrophies spinales pour les maladies où est impliquée la dégénérescence ou la mort des motoneurones de la moelle épinière.

La sclérose latérale amyotrophique (SLA ou ALS pour Amyotrophic Lateral Sclerosis) est une maladie neurodégénérative associée à différents types d'inclusions tels les corps de Lewis et caractérisée par une dégénérescence des motoneurones spinaux et corticaux dont l'issue fatale est parfois associée à une démence frontale. Au cours du développement de l'ALS, les phénomènes dégénératifs se produisent non seulement dans le cerveau mais également dans la moelle épinière et en conséquence dans le muscle, par défaut d'innervation.

On recherche toujours des composés actifs pour lutter contre les affections évoquées ci-dessus. Or la demanderesse a découvert que des dérivés de l'oxime de cholest-4-èn-3-one, et notamment la 3-oxyimino-cholest-4-èn-6-one étaient doués de remarquables propriétés neuroprotectrices et/ou cardioprotectrices. Les demandes de brevet WO 2004/082581, FR 2 852 246, FR 2 860 159 et ES 2 190 348 décrivent des dérivés du cholest-4-èn-3-one dont des oximes et décrivent leurs propriétés neuroprotectrices.

Dès lors, les intéressantes propriétés neuroprotectrices et cardioprotectrices des composés de formule I justifient leur utilisation comme médicament, particulièrement pour la préparation d'un médicament cytoprotecteur, très particulièrement neuroprotecteur ou cardioprotecteur.

Le terme "cytoprotecteur" fait référence à la capacité d'agents, par exemple des composés chimiques, naturels ou non, à maintenir les interactions des cellules entre elles ou avec les autres tissus, à protéger les cellules contre les phénomènes de dégénérescence conduisant à une perte de fonction cellulaire ou à des activités cellulaires indésirables, avec ou sans mort cellulaire, et/ou contre les dysfonctionnements cellulaires et/ou contre les maladies ou affections dégénératives conduisant à ces dysfonctionnements cellulaires, lesdits dysfonctionnements ou lesdites maladies ou affections conduisant ou non à la mort cellulaire.

Les termes "neuroprotecteur" ou cardioprotecteur" font référence aux mêmes propriétés desdits agents mais spécifiquement pour les cellules du système nerveux ("neuroprotecteur") soit spécifiquement pour les cellules du système cardiaque ("cardioprotecteur").

On comprend donc qu'un composé cytoprotecteur ou neuroprotecteur ou cardioprotecteur est un composé qui présente les propriétés précédemment décrites. C'est pourquoi la présente invention a pour objet l'utilisation à titre de médicaments des composés répondant à la formule I 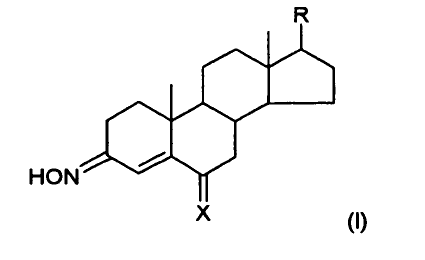 dans laquelle
X représente un atome d'oxygène ou un groupement =N-OH
R représente un groupement choisi parmi 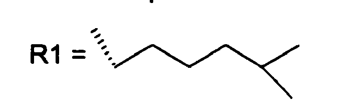 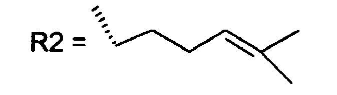 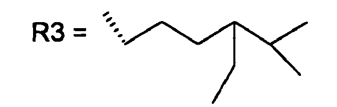 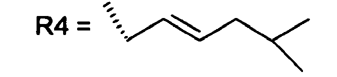 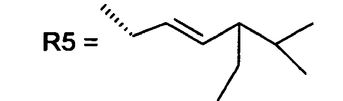 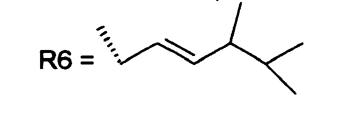
ainsi que leurs esters, et/ou leurs sels d'addition avec les acides pharmaceutiquement acceptables.

Les sels d'addition avec les acides pharmaceutiquement acceptables peuvent être par exemple des sels formés avec les acides chlorhydrique, bromhydrique, nitrique, sulfurique, phosphorique acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques, ou carboxyliques.

Ainsi, l'invention a aussi pour objet l'utilisation à titre de médicament de la cholest-4-èn-3,6-dioxime, la cholest-4,22-dièn-3,6-dioxime, la cholest-4,24-dièn-3,6-dioxime, la 3-oxyimino-cholest-4-èn-6-one, la 3-oxyimino-cholest-4,22-dièn-6-one, la 3-oxyimino-cholest-4,24-dièn-6-one, la 24-méthyl-3-oxyimino-cholest-4,22-dièn-6-one, la 24-méthyl-cholest-4,22-dièn-3,6-dioxime, la 24-éthyl-cholest-4-èn-3,6-dioxime, la 24-éthyl-cholest-4,22-dièn-3,6-dioxime, la 24-éthyl-3-oxyimino-cholest-4-èn-6-one, la 24-éthyl-3-oxyimino-cholest-4,22-dièn-6-one, ainsi que leurs esters, et/ou leurs leurs sels d'addition avec les acides pharmaceutiquement acceptables.

Parmi les composés ci-dessus décrits dont l'utilisation comme médicament est préférentielle, on retient notamment les composés de formule I pour lesquels X représente un atome d'oxygène, c'est-à-dire la 3-oxyimino-cholest-4-èn-6-one, la 3-oxyimino-cholest-4,22-dièn-6-one, la 3-oxyimino-cholest-4,24-dièn-6-one, la 24-méthyl-3-oxyimino-cholest-4,22-dièn-6-one, la 24-éthyl-3-oxyimino-cholest-4-èn-6-one, la 24-éthyl-3-oxyimino-cholest-4,22-dièn-6-one, ainsi que leurs esters, et/ou leurs sels d'addition avec les acides pharmaceutiquement acceptables.

On retient plus particulièrement la 3-oxyimino-cholest-4-èn-6-one et la 24-éthyl-3-oxyimino-holest-4-èn-6-one, ainsi que leurs esters, et/ou leurs sels d'addition avec les acides pharmaceutiquement acceptables.

On retient tout particulièrement la 3-oxyimino-cholest-4-èn-6-one ainsi que ses esters et/ou ses sels d'addition avec les acides pharmaceutiquement acceptables.

Les composés objet de la présente invention possèdent de très intéressantes propriétés pharmacologiques. Ils sont doués notamment de remarquables propriétés cytoprotectrices, particulièrement neuroprotectrices, très particulièrement vis à vis des motoneurones, et cardioprotectrices.

Ces propriétés sont illustrées ci-après dans la partie expérimentale. Elles justifient l'utilisation des composés ci-dessus décrits ainsi que celle de leurs esters et/ou de leurs sels d'addition avec les acides pharmaceutiquement acceptables à titre de médicament cytoprotecteurs, particulièrement de médicaments neuroprotecteurs et/ou cardioprotecteurs. Très particulièrement, les composés selon l'invention présentent une activité remarquable vis à vis des motoneurones, des neurones du système nerveux central, des nerfs moteurs et périphériques.

Dans le contexte de l'invention, le terme « traitement » désigne le traitement préventif, curatif, palliatif, ainsi que la prise en charge des patients (réduction de la souffrance, amélioration de la durée de vie, ralentissement de la progression de la maladie), etc. Le traitement peut en outre être réalisé en combinaison avec d'autres ingrédients ou traitements, tels que notamment d'autres composés actifs pour traiter les pathologies ou traumatismes spécifiés dans la présente demande.

Les composés selon la présente invention, en raison de leurs propriétés cytoprotectrices, peuvent être utilisés pour la préparation d'un médicament destiné au traitement ou à la prévention de la nécrose et/ou de l'apoptose pathologique et/ou de la nécroptose (médicaments antinécrotiques et/ou antiapoptotiques et/ou antinécroptotique) ou encore au traitement ou à la prévention des affections comme :
■ les maladies des os, des articulations, du tissu conjonctif et du cartilage, telles que l'ostéoporose, l'ostéomyélite, les arthrites dont par exemple l'ostéoarthrite, l'arthrite rhumatoïde et l'arthrite psoriasique, la nécrose avasculaire, la fibrodysplasie ossifiante progressive, le rachitisme, le syndrome de Cushing ;
■ les maladies musculaires telles que la dystrophie musculaire, comme par exemple la dystrophie musculaire de Duchenne, les dystrophies myotoniques, les myopathies et les myasthénies ;
■ les maladies de la peau, telles que les dermatites, l'eczéma, le psoriasis, le vieillissement, ou encore les altérations de la cicatrisation ;
■ les maladies cardiovasculaires telles que l'ischémie cardiaque et/ou vasculaire, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, l'anoxie, l'hypoxie, les effets secondaires dus à des thérapies avec des agents anticancéreux ;
■ les maladies circulatoires telles que l'athérosclérose, les scléroses artérielles, les maladies vasculaires périphériques, les accidents vasculaires cérébraux, les anévrismes ;
■ les maladies hématologiques et vasculaires telles que : l'anémie, l'amyloïdose vasculaire, les hémorragies, la drépanocytose, le syndrome de la fragmentation des globules rouges, la neutropénie, la leucopénie, l'aplasie médullaire, la pancytopénie, la thrombocytopénie, l'hémophilie ;
■ les maladies du poumon incluant la pneumonie, l'asthme ; les maladies chroniques obstructives des poumons comme par exemple les bronchites chroniques et l'emphysème ;
■ les maladies du tractus gastro-intestinal, telles que les ulcères ;
■ les maladies du foie incluant les hépatites virales et les cirrhoses, les maladies du foie dues à des toxines ou des médicaments ;
■ les maladies du pancréas comme par exemple les pancréatites aigues
ou chroniques ;
■ les maladies métaboliques telles que les diabètes mellitus et insipide, les thyroïdites ;
■ les maladies des reins telles que par exemple les désordres rénaux aigus ou la glomérulonéphrite ;
■ les infections virales et bactériennes telle que la septicémie ;
■ les intoxications sévères par des agents chimiques, des toxines ou des médicaments ;
■ les affections dégénératives associées au Syndrome Immuno Déficitaire Acquis (SIDA) ;
■ les désordres associés au vieillissement, tel que le syndrome du vieillissement accéléré ;
■ les maladies inflammatoires, telles que la maladie de Crohn, la polyarthrite rhumatoïde ;
■ les maladies auto-immunes telles que le lupus érythémateux ;
■ les désordres dentaires tels que ceux aboutissant à la dégradation des tissus comme par exemple les périodontites ;
■ les maladies ou désordres ophtalmiques incluant les rétinopathies diabétiques, le glaucome, les dégénérescences maculaires, la dégénérescence rétinienne, la rétinite pigmentaire, les trous ou déchirures rétiniennes, le décollement rétinien, l'ischémie rétinienne, les rétinopathies aigues associées à un traumatisme, les dégénérescences inflammatoires, les complications post chirurgicales, les rétinopathies médicamenteuses, la cataracte ;
■ les désordres des voies auditives, tels que l'otosclérose et la surdité induite par des antibiotiques ;
■ les maladies associées aux mitochondries (pathologies mitochondriales), telles que l'ataxie de Friedrich, la dystrophie musculaire congénitale avec anomalie mitochondriale structurelle, certaines myopathies (syndrome des MELAS, syndrome de MERFF, syndrome de Pearson), le syndrome de MIDD (diabètes mitochondriaux et surdité), le syndrome de Wolfram, la dystonie.

Très particulièrement, les médicaments selon la présente invention trouvent leur emploi en raison de leurs propriétés neuroprotectrices par exemple dans le traitement ou à la prévention des affections neurodégénératives, comme par exemple la maladie de Huntington, les maladies chroniques neurodégénératives, héréditaires ou sporadiques, les lésions neuronales liées au vieillissement, les neuropathies périphériques, héréditaires ou lésionnelles, les neuropathies diabétiques ou induites par les traitements anticancéreux, les traumatismes du cerveau, des nerfs périphériques ou de la moelle épinière, les ischémies du cerveau ou de la moelle épinière, les épilepsies, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, et notamment les amyotrophies spinales, la sclérose latérale amyotrophique et les pathologies dues aux traumatismes de la moelle épinière ou des nerfs moteurs périphériques.

Ils trouvent notamment en raison de leurs propriétés neuroprotectrices vis à vis des motoneurones, leur emploi particulièrement dans le traitement des amyotrophies spinales, notamment de la sclérose latérale amyotrophique ou des amyotrophies spinales infantiles, et dans le traitement des traumatismes de la moelle épinière ou des nerfs moteurs périphériques comme évoqué ci-dessus.

En général la dose journalière du composé sera la dose minimum pour obtenir l'effet thérapeutique. Cette dose dépendra des différents facteurs cités auparavant. Les doses des composés ci-dessus décrits et par exemple de la 3-oxyimino-cholest-4-èn-6-one seront en général comprises entre 0,001 à 100 mg par kilo par jour pour l'homme.

Si nécessaire, la dose journalière peut être administrée en deux, trois, quatre, cinq, six ou plus, prises par jour ou par sous-doses multiples administrées par intervalles appropriés pendant la journée.

La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.

La prescription du médecin traitant pourra commencer à des doses inférieures à celles généralement utilisées, puis ces doses seront progressivement augmentées afin de mieux maîtriser l'apparition d'éventuels effets secondaires.

L'invention a aussi pour objet les compositions pharmaceutiques qui renferment au moins un composé précité ou un de ses esters et/ou de ses sels d'addition avec les acides pharmaceutiquement acceptables, à titre de principe actif.

Dans ces compositions, le principe actif est avantageusement présent à des doses physiologiquement efficaces ; les compositions précitées renferment notamment une dose neuroprotectrice efficace d'au moins un principe actif ci-dessus.

A titre de médicaments, les composés répondant à la formule I ainsi que leurs esters et/ou leurs sels d'addition avec les acides pharmaceutiquement acceptables peuvent être incorporés dans des compositions pharmaceutiques destinées à la voie digestive ou parentérale.

Les compositions pharmaceutiques selon l'invention peuvent comprendre en outre au moins un autre ingrédient thérapeutiquement actif, pour une utilisation simultanée, séparée ou étalée dans le temps, notamment lors d'un traitement chez un sujet atteint d'une pathologie ou d'un traumatisme lié à la dégénérescence ou à la mort des cellules particulièrement des cellules cardiaques et/ou des motoneurones tel que défini ci-dessus.

Les compositions pharmaceutiques ou médicaments selon l'invention comprennent avantageusement un ou plusieurs excipients ou véhicules inertes, ) c'est à dire pharmaceutiquement inactifs et non toxiques. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, les cyclodextrines, le polysorbate 80, le mannitol, la gélatine, le lactose, des huiles végétales ou animales, l'acacia, etc. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

L'administration peut être réalisée par toute méthode connue de l'homme du métier, de préférence par voie orale ou par injection, typiquement par voie intra-péritonéale, intra-cérébrale, intra-thécale, intra-veineuse, intra-artérielle ou intra-musculaire. L'administration par voie orale est préférée. S'agissant d'un traitement à long terme, la voie d'administration préférée sera sublinguale, orale ou transcutanée.

Pour les injections, les composés sont généralement conditionnés sous forme de suspensions liquides, qui peuvent être injectées au moyen de seringues ou de perfusions, par exemple. Il est entendu que le débit et/ou la dose injectée, ou de manière générale la dose à administrer, peuvent être adaptés par l'homme du métier en fonction du patient, de la pathologie, du mode d'administration, etc.. Il est entendu que des administrations répétées peuvent être réalisées, éventuellement en combinaison avec d'autres ingrédients actifs ou tout véhicule acceptable sur le plan pharmaceutique (tampons, solutions saline, isotonique, en présence d'agents stabilisants, etc.).

L'invention est utilisable chez les mammifères, notamment chez l'être humain.

La présente invention a encore pour objet un procédé de préparation d'une composition ci-dessus décrite, **caractérisé en ce que** l'on mélange, selon des méthodes connues en elles mêmes, le ou les principes actifs avec des excipients acceptables, notamment pharmaceutiquement acceptables.

Certains des composés de formule I tels que définis ci-dessus sont connus ou peuvent être préparés selon des procédés décrits dans la littérature. Mais certains dérivés de formule I sont des produits nouveaux.

C'est pourquoi la présente demande a aussi pour objet des composés nouveaux répondant à la formule I 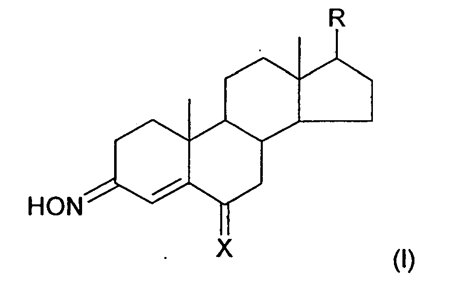 dans laquelle X représente un atome d'oxygène ou un groupement =N-OH et R représente un groupement choisi parmi 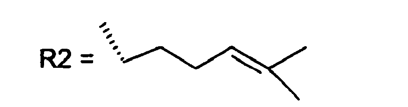 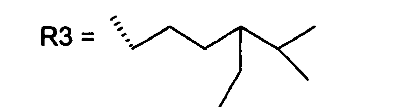 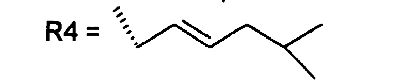 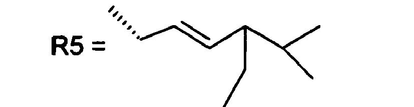 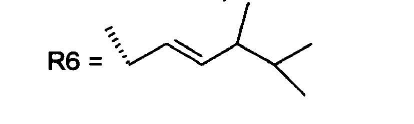 ainsi que leurs esters et/ou leurs sels d'addition avec les acides minéraux ou organiques.

Ainsi l'invention a pour objet un composé répondant à la formule I ci-dessus, choisi parmi la cholest-4,22-dièn-3,6-dioxime, la cholest-4,24-dièn-3,6-dioxime, la 3-oxyimino-cholest-4,22-dièn-6-one, la 3-oxyimino-cholest-4,24-dièn-6-one, la 24-méthyl-3-oxyimino-cholest-4,22-dièn-6-one, la 24-éthyl-3-oxyimino-cholest-4,22-dièn-6-one, la 24-éthyl-3-oxyimino-cholest-4-èn-6-one, la 24-méthyl-cholest-4,22-dièn-3,6-dioxime, la 24-éthyl-cholest-4-èn-3,6-dioxime, ainsi que leurs esters, et/ou leurs sels d'addition avec les acides pharmaceutiquement acceptables.

Parmi les composés ci-dessus décrits on retient notamment les composés de formule I pour lesquels X représente un atome d'oxygène ainsi que leurs esters et/ou leurs sels d'addition avec les acides pharmaceutiquement acceptables, c'est-à-dire la 3-oxyimino-cholest-4,22-dièn-6-one, la 3-oxyimino-cholest-4,24-dièn-6-one, la 24-méthyl-3-oxyimino-cholest-4,22-dièn-6-one, la 24-éthyl-3-oxyimino-cholest-4-èn-6-one, la 24-éthyl-3-oxyimino-cholest-4,22-dièn-6-one, ainsi que leurs esters, et/ou leurs sels d'addition avec les acides pharmaceutiquement acceptables.

On retient plus particulièrement la 3-oxyimino-cholest-4,24-dièn-6-one et la 24-éthyl-3-oxyimino-cholest-4-èn-6-one, ainsi que leurs esters, et/ou leurs sels d'addition avec les acides pharmaceutiquement acceptables et encore plus particulièrement la 3-oxyimino-cholest-4,24-dièn-6-one ainsi que ses esters et/ou ses sels d'addition avec les acides pharmaceutiquement acceptables.

La présente invention a également pour objet un procédé de préparation des nouveaux composés de formule I tels que définis ci-dessus ainsi que de leurs esters, et/ou leurs sels, **caractérisé en ce que** l'on fait réagir un composé de formule II 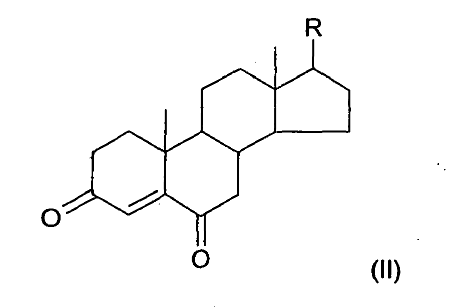 dans laquelle
R représente un groupement choisi parmi 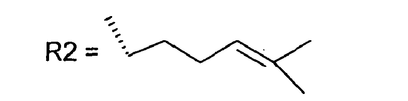 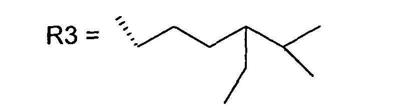 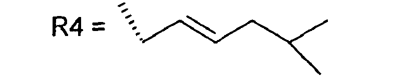 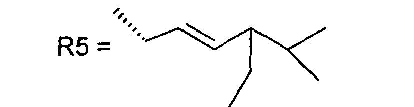 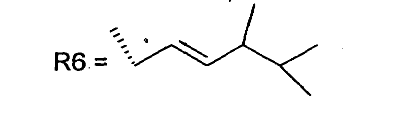 avec un halogénure d'hydroxylamine comme le chlorhydrate d'hydroxylamine, pour obtenir le composé de formule I attendu que l'on isole et si désiré salifie.

Dans des conditions préférentielles de mise en oeuvre du procédé ci-dessus décrit,
- on solubilise le produit de départ dans un minimum d'un solvant adapté comme par exemple la pyridine,
- on utilise soit
   ○ 1 équivalent d'halogénure d'hydroxylamine, afin d'obtenir majoritairement les composés 3-oxyimino -6-one ;
   ○ un excès d'halogénure d'hydroxylamine, afin d'obtenir majoritairement les composés 3,6-dioxime ;
- on opère sous agitation pendant environ 24h à température ambiante (20-30°C).

Les composés de formule II sont des dérivés connus, dont la synthèse est décrite dans la littérature.

L'invention a encore pour objet l'utilisation d'un composé de formule I 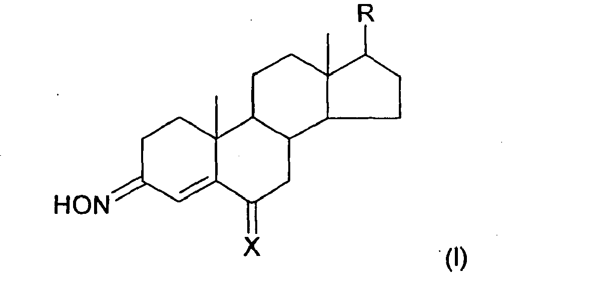 dans laquelle X représente un atome d'oxygène ou un groupement = N-OH, et R représente un groupement choisi parmi 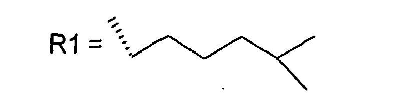 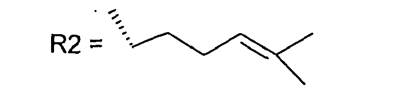 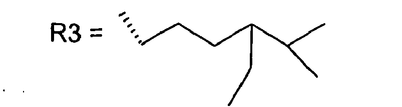 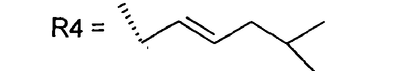 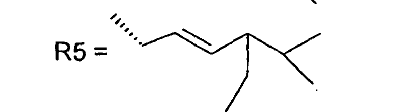 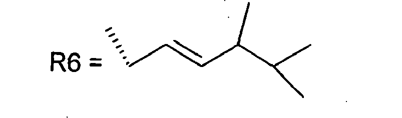 ou de l'un de ses esters, et/ou de ses sels d'addition avec les acides pharmaceutiquement acceptables, pour la préparation d'un médicament cytoprotecteur.

L'invention a particulièrement pour objet l'utilisation d'un composé de formule I ci-dessus dans la préparation d'un médicament destiné au traitement ou à la prévention des pathologies ou traumatismes liés à la dégénérescence ou à la mort des cellules, particulièrement des cellules cardiaques et/ou des neurones, que celles-ci soient naturelles ou accidentelles.

L'invention a encore plus particulièrement pour objet l'utilisation d'un composé de formule I ci-dessus dans la préparation d'un médicament destiné au traitement ou à la prévention de la nécrose et/ou de l'apoptose pathologique et/ou de la nécroptose (médicaments antinécrotiques et/ou antiapoptotiques et/ou antinécroptotiques) ou encore au traitement ou à la prévention des affections comme :
■ les maladies des os, des articulations, du tissu conjonctif et du cartilage, telles que l'ostéoporose, l'ostéomyélite, les arthrites dont par exemple l'ostéoarthrite, l'arthrite rhumatoïde et l'arthrite psoriasique, la nécrose avasculaire, la fibrodysplasie ossifiante progressive, le rachitisme, le syndrome de Cushing ;
■ les maladies musculaires telles que la dystrophie musculaire, comme par exemple la dystrophie musculaire de Duchenne, les dystrophies myotoniques, les myopathies et les myasthénies ;
■ les maladies de la peau, telles que les dermatites, l'eczéma, le psoriasis, le vieillissement, ou encore les altérations de la cicatrisation;
■ les maladies cardiovasculaires telles que l'ischémie cardiaque et/ou vasculaire, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, l'anoxie, l'hypoxie, les effets secondaires dus à des thérapies avec des agents anticancéreux ;
■ les maladies circulatoires telles que l'athérosclérose, les scléroses artérielles, et les maladies vasculaires périphériques, les accidents vasculaires cérébraux, les anévrismes ;
■ les maladies hématologiques et vasculaires telles que : l'anémie, l'amyloïdose vasculaire, les hémorragies, la drépanocytose, le syndrome de la fragmentation des globules rouges, la neutropénie, la leucopénie, l'aplasie médullaire, la pancytopénie, la thrombocytopénie, l'hémophilie ;
■ les maladies du poumon incluant la pneumonie, l'asthme ; les maladies chroniques obstructives des poumons comme par exemple les bronchites chroniques et l'emphysème ;
■ les maladies du tractus gastro-intestinal, telles que les ulcères ;
■ les maladies du foie incluant les hépatites virales et les cirrhoses, les maladies du foie dues à des toxines ou des médicaments ;
■ les maladies du pancréas comme par exemple les pancréatites aigues
ou chroniques ;
■ les maladies métaboliques telles que les diabètes mellitus et insipide, les thyroïdites ;
■ les maladies des reins telles que par exemple les désordres rénaux aigus ou la glomérulonéphrite ;
■ les infections virales et bactériennes telle que la septicémie ;
■ les intoxications sévères par des agents chimiques, des toxines ou des médicaments ;
■ les affections dégénératives associées au Syndrome Immuno Déficitaire Acquis (SIDA) ;
■ les désordres associés au vieillissement, tel que le syndrome du vieillissement accéléré ;
■ les maladies inflammatoires, telles que la maladie de Crohn, la polyarthrite rhumatoïde ;
■ les maladies auto-immunes telles que le lupus érythémateux ;
■ les désordres dentaires tels que ceux aboutissant à la dégradation des tissus comme par exemple les périodontites ;
■ les maladies ou désordres ophtalmiques incluant les rétinopathies diabétiques, le glaucome, les dégénérescences maculaires, la dégénérescence rétinienne, la rétinite pigmentaire, les trous ou déchirures rétiniennes, le décollement rétinien, l'ischémie rétinienne, les rétinopathies aigues associées à un traumatisme, les dégénérescences inflammatoires, les complications post chirurgicales, les rétinopathies médicamenteuses, la cataracte ;
■ les désordres des voies auditives, tels que l'otosclérose et la surdité induite par des antibiotiques ;
■ les maladies associées aux mitochondries (pathologies mitochondriales), telles que l'ataxie de Friedrich, la dystrophie musculaire congénitale avec anomalie mitochondriale structurelle, certaines myopathies (syndrome des MELAS, syndrome de MERFF, syndrome de Pearson), le syndrome de MIDD (diabètes mitochondriaux et surdité), le syndrome de Wolfram, la dystonie, et particulièrement
■ des maladies neurodégénératives comme par exemple la maladie de Huntington, les maladies chroniques neurodégénératives, héréditaires
ou sporadiques, les lésions neuronales liées au vieillissement, les neuropathies périphériques héréditaires ou lésionnelles, la maladie de Charcot-Marie-Tooth, les neuropathies diabétiques ou induites par les traitements anticancéreux, les épilepsies, les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, les maladies et traumatismes liés à la dégénérescence des motoneurones et plus particulièrement les amyotrophies spinales particulièrement infantiles, la sclérose latérale amyotrophique, la sclérose en plaques et les traumatismes de la moelle épinière ou des nerfs moteurs périphériques.

L'invention a tout particulièrement pour objet l'utilisation d'un composé de formule I dans la préparation d'un médicament destiné au traitement des amyotrophies spinales, particulièrement infantiles, et des scléroses latérales amyotrophiques.

La mise en oeuvre de ces médicaments comprend habituellement l'administration aux patients, particulièrement aux mammifères, tout particulièrement aux êtres humains, d'une quantité thérapeutiquement efficace d'un composé de formule I et notamment de la 3-oxyimino-cholest-4-èn-6-one, en particulier pour augmenter la survie des cellules, particulièrement des cellules cardiaques et/ou des neurones ou favoriser la croissance axonale.

L'invention a tout autant pour objet une méthode de traitement des maladies précitées, notamment neurodégénératives, et notamment une méthode de traitement de pathologies ou traumatismes liés à la dégénérescence ou à la mort des neurones, chez des mammifères (en général des patients) atteints de telles pathologies ou traumatismes, comprenant l'administration à ces mammifères d'une quantité thérapeutiquement efficace de 3-oxyimino-cholest-4-èn-6-one, en particulier pour augmenter la survie des neurones ou favoriser la croissance axonale.

L'invention a de plus pour objet une méthode de traitement d'une des affections ci-dessus décrites et notamment des pathologies ou traumatismes liés à la dégénérescence ou à la mort des motoneurones, chez des mammifères (en général des patients) atteints de tels pathologies ou traumatismes, comprenant l'administration à ces mammifères d'une quantité thérapeutiquement efficace d'un composé de formule I, en particulier pour augmenter la survie des neurones. Plus spécifiquement, les pathologies liées à la dégénérescence ou à la mort des motoneurones sont la sclérose latérale amyotrophique ou les amyotrophies spinales infantiles.

Les exemples qui suivent illustrent l'invention sans la limiter.

### EXEMPLE 1

On a préparé une suspension répondant à la formule

| | |
|---|---|
| 3-oxyimino-cholest-4-èn-6-one | 20 mg par ml |
| Excipient : | acide oléique |
| Conservateur : | méthylparaben |

### EXEMPLE 2

On a préparé des gélules molles répondant à la formule

| | |
|---|---|
| 3-oxyimino-cholest-4-èn-6-one | 250 mg |
| Excipient : quantité suffisante pour une gélule terminée à | 750 mg |

### EXEMPLE 3 : Préparation de la 3-oxyimino-cholest-4-èn-6-one (R = R1)

Dans un ballon de 250 ml, on solubilise 2,8 g de cholest-4-èn-3,6-dione (7,03 mmol) dans 90 ml de pyridine à 0°C, puis on ajoute 489 mg (7,03 mmol) de chlorhydrate d'hydroxylamine. La solution est agitée pendant 12 heures en laissant la température remonter progressivement à température ambiante. On ajoute une solution d'HCl 1M, puis de l'éther diéthylique afin d'effectuer une extraction. Les phases organiques sont séchées sur MgS04. Après filtration, l'éther diéthylique est évaporé sous pression réduite. La purification est réalisée par chromatographie flash (25/75 éther diéthylique/éther de pétrole) sur gel de silice suivi d'un lavage du solide obtenu avec de l'éther diisopropylique. On obtient la 3-oxyimino-cholest-4-èn-6-one sous forme d'un solide blanc (530 mg, 1,28 mmol, 18%). Rf = 0,58 (70/30 (éther diéthylique/éther de pétrole)).

Analyses :
- Chromatographie Liquide / Spectrométrie de Masse (Electrospray®)
- Conditions de la chromatographie liquide haute performance :
   Colonne : Thermo- Hypersil Hyperprep - RP C18 8 µm- 150x4,6 mm
   Gradient : eau (+0.05% TFA) / acétonitrile (+0,05% TFA)
      t = 0 min : 80% acétonitrile, 20% H₂O
      t = 15 min : 95% acétonitrile, 5% H₂O
      t = 27 min : 95% acétonitrile, 5% H₂O
   Temps de rétention : 20.32 min (unité en 100^{ème} de min)
   Pic détecté en spectrométrie de masse : {M+H}⁺ = 414

### EXEMPLES 4 à 8 :

On prépare selon un protocole identique à celui utilisé à l'exemple 3, les composés suivants

| Exemple n° | Composé préparé | Composé de départ |
|---|---|---|
| 4 (R=R2) | 3-oxyimino-cholest-4,24-dièn-6-one | cholest-4,24-dièn-3,6-dione |
| 5 (R=R3) | 24-éthyl-3-oxyimino-cholest-4-èn-6-one | 24-éthyl-cholest-4-èn-3,6-dione |
| 6 (R=R4) | 3-oxyimino-cholest-4,22-dièn-6-one | cholest-4,22-dièn-3,6-dione |
| 7 (R=R5) | 24-éthyl-3-oxyimino-cholest-4,22-dièn-6-one | 24-éthyl-cholest-4,22-dièn-3,6-dione |
| 8 (R=R6) | 24-méthyl-3-oxyimino-cholest-4,22-dièn-6-one | 24-méthyl-cholest-4,22-dièn-3,6-dione |

### EXEMPLE 9 : Préparation de la cholest-4-èn-3,6-dioxime (R = R1)

Dans un ballon de 250 ml, on solubilise 2,8 g de cholest-4-èn-3,6-dione (7,03 mmol) dans 90 ml de pyridine à 0°C, puis on ajoute 1467mg (21,09 mmol) de chlorhydrate d'hydroxylamine. La solution est agitée pendant 12 heures en laissant la température remonter progressivement à température ambiante. On ajoute une solution d'HCl 1M, puis de l'éther diéthylique afin d'effectuer une extraction. Les phases organiques sont séchées sur MgS04. Après filtration, l'éther diéthylique est évaporé sous pression réduite. La purification est réalisée par chromatographie flash (40/60 éther diéthylique/éther de pétrole) sur gel de silice. Le solide obtenu (778 mg) est recristallisé dans l'éther diisopropylique. On obtient la cholest-4-èn-3,6-dioxime sous forme d'un solide blanc (487 mg, 1,14 mmol, 16%). Rf = 0,21(70/30 (éther diéthylique/éther de pétrole)).

Analyses :
- Chromatographie Liquide / Spectrométrie de Masse (Electrospray®)
- Conditions de la chromatographie liquide haute performance :
   Colonne : Thermo- Hypersil Hyperprep - RP C18 8 µm- 150x4,6 mm
   Gradient : eau (+0,05% TFA) / acétonitrile (+0,05% TFA)
      t = 0 min : 80% acétonitrile, 20% H₂O
      t = 15 min : 95% acétonitrile, 5% H₂O
      t = 27 min : 95% acétonitrile, 5% H₂O
   Temps de rétention : 15,71 min et 17,61 min (unité en 100^{ème} de min)
   Pic détecté en spectrométrie de masse : {M+H}⁺ = 429

### EXEMPLES 10 à 14 :

On prépare selon un protocole identique à celui utilisé à l'exemple 9, les composés suivants

| Exemple n° | Composé préparé | Composé de départ |
|---|---|---|
| 10 (R=R2) | cholest-4,24-dièn-3,6-dioxime | cholest-4,24-dièn-3,6-dione |
| 11 (R=R3) | 24-éthyl-cholest-4-èn-3,6-dioxime | 24-éthyl-cholest-4-èn-3,6-dione |
| 12 (R=R4) | cholest-4,22-dièn-3,6-dioxime | cholest-4,22-dièn-3,6-dione |
| 13 (R=R5) | 24-ethyl-cholest-4,22-dièn-3,6-dioxime | 24-éthyl-cholest-4,22-dièn-3,6-dione |
| 14 (R=R6) | 24-méthyl-cholest-4,22-dièn-3,6-dioxime | 24-méthyl-cholest-4,22-dièn-3,6-dione |

### ETUDE PHARMACOLOGIQUE

Les composes ont été testés selon les protocoles suivants :

### EXEMPLE 15 : Effets des composés de formule I sur la survie des motoneurones

Pour mettre en évidence l'action neuroprotectrice des composés de formule I, la demanderesse a étudié leur activité sur un modèle *in vitro* de privation trophique de motoneurones de rats. On pourra se référer utilement à la demande de brevet WO 0142784 de la demanderesse sur la mise en culture des motoneurones de moelle épinière.

La moelle épinière d'embryons E14 de rat est disséquée et la partie ventrale est dissociée par trituration après trypsination. Les motoneurones sont séparés des autres cellules spinales par une méthode connue (Camu et al, 1993, Purification of spinal motoneurons from chicken and rat embryos by immunopanning. In « Immunoselection Strategies for Neural cell culture », Neuroprotocols : A companion to Methods in Neurosciences 2, 191-199 ; Henderson et al., 1993, Neutrophins promote motor neuron survival and are present in embryonic limb bud. Nature 363 (6426) :266-70). Les cellules sont centrifugées sur un gradient de densité. Les motoneurones sont enrichis dans la fraction des grandes cellules (les moins denses). Les cellules de cette fraction sont incubées avec un anticorps anti-p75, un antigène de surface présent sur les motoneurones. Des anticorps secondaires couplés à des billes magnétiques sont ajoutés et le mélange de cellules est passé à travers une colonne dans un aimant (Arce et al, 1999 Cardiotrophin-1 requires LIFRbeta to promote survival of mouse motoneurons purified by a novel technique. J. Neurosci Res 55(1): 119-26). Seuls les motoneurones sont retenus : leur pureté est de l'ordre de 90%.

Les motoneurones sont ensemencés à faible densité dans des puits de culture sur un substrat de polyornithine-laminine dans un milieu neurobasal (GIBCO) supplémenté selon Raoul et al, 1999, Programmed cell death of embryonic motoneurons triggered through the Fas death receptor. J Cell Biol 147(5) :1049-62. Des contrôles négatifs (absence de facteurs trophiques) et positifs (en présence de BDNF (Brain-Derived Neurotrophic Factor) à 1 ng/ml, GDNF (Glial-Derived Neurotrophic Factor) à 1 ng/ml et CNTF (Ciliary Neurotrophic Factor) à 10 ng/ml, commercialisés par la société américaine PEPROTECH, Inc. et la société Sigma-Aldrich, sont inclus dans chaque série.

Les composés à tester sont ajoutés 60 minutes après l'ensemencement et les cultures sont maintenues à 37°C sous 5% de CO₂ pendant 3 jours.

Les motoneurones ont une tendance spontanée à mourir en l'absence de facteurs neurotrophiques (Pettmann et Henderson, 1998, Neuronal cell death. Neuron 20(4):633-47). Après 3 jours, la survie est évaluée par une mesure de fluorescence après incubation des cellules en présence de calcéine qui devient fluorescente dans les cellules vivantes.

Après 3 jours en culture à 37°C, sous 5% de CO₂ et en humidité saturante, jusqu'à 50% des motoneurones ensemencés initialement survivent dans le milieu supplémenté en facteurs neurotrophiques, alors que moins de 15% des motoneurones survivent en milieu de culture non additionné de facteurs neurotrophiques..

L'activité neuroprotectrice des composés à tester a été évaluée par leur capacité à empêcher la mort des motoneurones quand ils sont ajoutés au milieu Neurobasal (GIBCO) en comparaison avec la survie des motoneurones en milieu additionné de facteurs neurotrophiques.

Les composés de formule I selon l'invention ont montré une activité neuroprotectrice à une concentration capable de permettre un meilleur taux de survie des motoneurones dans le milieu Neurobasal. Ce taux de survie est exprimé par le nombre de cellules vivantes après traitement avec le composé à tester par rapport à la survie induite par les facteurs neurotrophiques. Ce rapport représente donc le pourcentage de survie due au composé testé par rapport à la survie induite par les facteurs neurotrophiques. Si le rapport est supérieur à O, l'effet des composés est positif sur la survie des motoneurones.

Les résultats obtenus sont les suivants :

| Composé de l'exemple | Concentration en µM | Ratio |
|---|---|---|
| 3 | 3 | 0,60 |
| 7 | 1 | 0,46 |
| 9 | 0,6 | 0,25 |
| 10 | 1 | 0,25 |
| 13 | 1 | 0,34 |

De par leur effet trophique sur les motoneurones spinaux, les composés de formule I selon l'invention se montrent donc utiles comme médicament, notamment dans le traitement des amyotrophies, en particulier dans le traitement de la sclérose latérale amyotrophique ou des amyotrophies spinales infantiles, et dans le traitement des traumatismes de la moelle épinière.

### EXEMPLE 16 : Effets des composés de formule I sur la protection des neurones striataux de la mort induite par la surexpression d'une forme mutée de la huntingtine

Des cultures primaires de neurones striataux sont préparées comme décrit dans la littérature (Primary striatal neuronal culture, Mao L. et al., Methods Mol Med., 2003, 79:379-86). Les cellules sont électroporées d'après la procédure décrite par Raoul et al., (Motoneuron death triggered by a specific pathway downstream of Fas. potentiation by ALS-linked SOD1 mutations Neuron, 2002, 35:1067-83) avant l'ensemencement avec un vecteur ou plasmide d'expression contenant un élément promoteur suivi de l'ADN codant pour une forme tronquée de la huntingtine qui comprend les 480 premiers amino acides et 68 CAG (Saudou et al., Huntingtin acts in the nucleus to induce apoptosis but death does not correlate with the formation of intranuclear inclusions, Cell, 1998, 95:55-66). Un second vecteur d'expression contenant l'ADN codant la *green fluorescent protein* (GFP) est également électroporé et sert de gène rapporteur. L'ADN du plasmide codant la huntingtine a été préparé par purification au chlorure de césium. Le plasmide contenant la séquence GFP a été préparée sur colonnes Qiagen. L'intégrité des séquences d'ADN est vérifiée par séquençage, transfection et *western blotting*. Les cellules qui survivent à l'électroporation sont ensemencées à une densité de 4000 cellules par puits de plaques 96 puits. La culture se fait dans du milieu Neurobasal (GIBCO) complémenté avec du pyruvate et du B-27 (Beckton Dickinson). Les cellules sont maintenues en culture pendant 7 jours sans changer le milieu.

Les traitements avec les composés à tester se font juste après l'ensemencement à une concentration finale de 1 µM dans 0.5% de diméthylsulfoxyde (DMSO). Les contrôles positifs se font par adjonction de BDNF à 5 ng/ml final. Les contrôles négatifs ne reçoivent que 0.5% de DMSO.

La mort cellulaire est évaluée après les 7 jours par comptage du nombre de cellules vivantes exprimant la GFP.

L'activité des composés à tester a été évaluée par leur capacité à empêcher la mort des neurones striataux cultivés dans le milieu neurobasal en comparaison avec la survie des neurones striataux en milieu supplémenté avec du BDNF (Brain-Derived Neurotrophic Factor).

Les résultats obtenus sont les suivants :

| Composé de l'exemple | Concentrations en µM | Ratio |
|---|---|---|
| 3 | 1 | 0,3 |
| 9 | 0,3 | 0,5 |

A la concentration de 10⁻⁶ M, les composes à tester montrent un effet protecteur de la mort cellulaire induite par la huntingtine mutée jusqu'à 60 % par comparaison aux cellules traitées par le BDNF.

De par leur effet neuroprotecteur, les composés de formule I selon l'invention se montrent donc utiles comme médicaments destiné au traitement ou à la prévention des affections neurodégénératives, notamment dans le traitement des amyotrophies spinales, de la sclérose latérale amyotrophique, dans le traitement des traumatismes de la moelle épinière et des nerfs périphériques et dans le traitement de la maladie de Huntington.

### Exemple 17 : Effets des composés de formule I sur la protection des neurones corticaux.

Les neurones corticaux ont été préparés à partir d'embryons de rat Sprague-Dawley au stade E18 de gestation et cultivés en plaque 96 puits à une densité de 333 cellules/mm² en milieu Neurobasal (Invitrogen) supplémenté avec 2% de B27 (Invitrogen) et 2% de sodium pyruvate. Ces conditions de culture permettent d'obtenir une très bonne pureté des cultures en neurones corticaux. Après 6 jours en culture, la mort des neurones corticaux a été induite par l'ajout de 10 µM de camptothécine dissous dans du dimethyl sulfoxide (DMSO), Au même moment, les neurones ont été traités par différentes concentrations de composé à tester. La concentration finale en DMSO après traitement est de 1 %. Après 16 heures d'incubation avec la camptothécine, la survie des neurones a été évaluée par comptage des cellules survivantes. Pour ce faire, les cellules ont été incubées pendant 20 minutes en présence d'un colorant vital, la calcéine-AM (Invitrogen) à 2 µg/ml. Après marquage, chaque puits de culture a été analysé à l'aide d'une station d'imagerie (Flash Cytomètre ,TROPHOS, FRANCE) munie d'un logiciel d'analyse d'images. TINA 4.5 (TROPHOS, France) permettant une prise d'image numérisée de l'ensemble du puits (temps d'exposition 40 ms). Les cellules, définies par des critères de taille de pixel (min = 10 ; max = 40), ont été ensuite comptées à l'aide du logiciel Tina 4.5 (TROPHOS)

Les résultats obtenus sont les suivants :

La camptothécine induit une diminution de la viabilité cellulaire. L'incubation des cellules avec les composés à tester augmente de manière dose-dépendante la survie cellulaire avec une EC50 de l'ordre de la centaine de nM.

| | Contrôle | | Composé 3 | | |
|---|---|---|---|---|---|
| | | 0 | 0,1µM | 0,3µM | 1µM |
| Viabilité cellulaire | 100 ± | 43,2 ± | 63,3 ± | 99,1 ± | 112,2 ± |
| (%) | 18,4 | 17,4 | 20,3 | 15,7 | 22,3 |

| | | | | | |
|---|---|---|---|---|---|
| Moyenne ± écart type, n = 8 puits | | | | | |

## Revendications

1. Composé répondant à la formule I 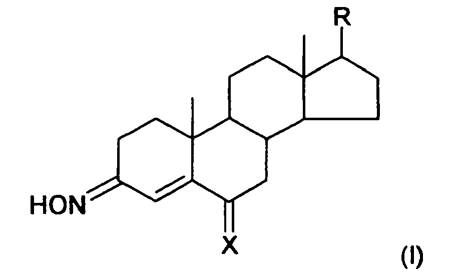 dans laquelle X représente un atome d'oxygène ou un groupement =N-OH, et
R représente un groupement choisi parmi 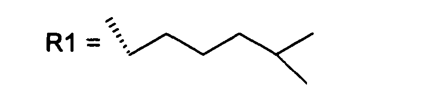 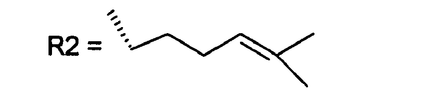 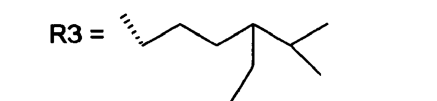 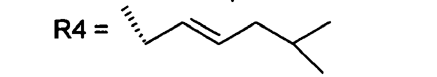 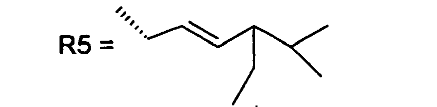 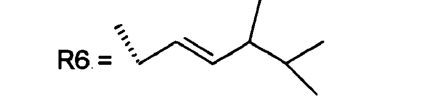 ou de l'un de ses esters et/ou de ses sels d'addition avec les acides pharmaceutiquement acceptables, pour son utilisation comme médicament.

2. Composé selon la revendication 1, **caractérisée en ce qu'**il est choisi parmi la 3-oxyimino-cholest-4-èn-6-one, la 3-oxyimino-cholest-4,24-dièn-6-one, la 24-éthyl-3-oxyimino-cholest-4-èn-6-one, la 3-oxyimino-cholest-4,22-dièn-6-one, la 24-éthyl-3-oxyimino-cholest-4,22-dièn-6-one, la 24-méthyl-3-oxyimino-cholest-4,22-dièn-6-one, la cholest-4-èn-3,6-dioxime, la cholest-4,24-dièn-3,6-dioxime, la 24-éthyl-cholest-4-èn-3,6-dioxime, la cholest-4,22-dièn-3,6-dioxime, la 24-éthyl-cholest-4,22-dièn-3,6-dioxime, la 24-méthyl-cholest-4,22-dièn-3,6-dioxime, ou de l'un de ses esters et/ou de ses sels d'addition avec les acides pharmaceutiquement acceptables.

3. Composé selon l'une quelconque des revendications 1 ou 2, **caractérisé en ce qu'**il est choisi parmi la 3-oxyimino-cholest-4-èn-6-one, la 3-oxyimino-cholest-4,24-dièn-6-one, la 24-éthyl-3-oxyimino-cholest-4-èn-6-one, la 3-oxyimino-cholest-4,22-dièn-6-one, la 24-éthyl-3-oxyimino-cholest-4,22-dièn-6-one, la 24-méthyl-3-oxyimino-cholest-4,22-dièn-6-one, ou de l'un de ses esters et/ou de ses sels d'addition avec les acides pharmaceutiquement acceptables.

4. Composé selon l'une quelconque des revendications 1 à 3, **caractérisé en ce qu'**il est choisi parmi la 3-oxyimino-cholest-4-èn-6-one ou la 24-éthyl-3-oxyimino-cholest-4-èn-6-one, particulièrement la 3-oxyimino-cholest-4-èn-6-one, ou de l'un de ses esters et/ou de ses sels d'addition avec les acides pharmaceutiquement acceptables.

5. Utilisation d'un composé de formule I tel que décrit dans l'une quelconque des revendications 1 à 4, pour la préparation d'un médicament cytoprotecteur, préférentiellement cardioprotecteur et/ou neuroprotecteur.

6. Utilisation d'un composé de formule I ci-dessus dans la préparation d'un médicament destiné au traitement ou à la prévention de la nécrose et/ou de l'apoptose pathologique et/ou de la nécroptose (médicaments antinécrotiques et/ou antiapoptotiques et/ou antinécroptotiques)

7. Utilisation d'un composé de formule I tel que décrit dans l'une quelconque des revendications 1 à 4, pour la préparation d'un médicament destiné au traitement des pathologies ou traumatismes liés à la dégénérescence ou à la mort des cellules, particulièrement des cellules cardiaques et/ou des neurones, que celles-ci soient naturelles ou accidentelles.

8. Utilisation d'un composé de formule I tel que décrit dans l'une quelconque des revendications 1 à 4, ou de l'un de ses esters et/ou de ses sels d'addition avec les acides pharmaceutiquement acceptables, pour la préparation d'un médicament destiné au traitement des affections comme :
■ les maladies des os, des articulations, du tissu conjonctif et du cartilage ;
■ les maladies musculaires telles que la dystrophie musculaire ;
■ les maladies de la peau ;
■ les maladies cardiovasculaires ;
■ les maladies circulatoires ;
■ les maladies hématologiques et vasculaires ;
■ les maladies du poumon dont les maladies chroniques obstructives des poumons ;
■ les maladies du tractus gastro-intestinal ;
■ les maladies du foie ;
■ les maladies du pancréas ;
■ les maladies métaboliques ;
■ les maladies des reins ;
■ les infections virales et bactériennes ;
■ les intoxications sévères ;
■ les affections dégénératives associées au Syndrome Immuno Déficitaire Acquis (SIDA) ;
■ les désordres associés au vieillissement ;
■ les maladies inflammatoires ;
■ les maladies auto-immunes ;
■ les désordres dentaires ;
■ les maladies ou désordres ophtalmiques ;
■ les désordres des voies auditives ;
■ les maladies associées aux mitochondries (pathologies mitochondriales).

9. Utilisation d'un composé de formule I tel que décrit dans l'une quelconque des revendications 1 à 4, ou de l'un de ses esters et/ou de ses sels d'addition avec les acides pharmaceutiquement acceptables, pour la préparation d'un médicament destiné au traitement des maladies neurodégénératives.

10. Utilisation selon la revendication 8 ou 9, **caractérisée en ce que** le médicament neuroprotecteur est destiné au traitement d'une maladie neurodégénérative choisie parmi la maladie de Huntington, les maladies chroniques neurodégénératives, héréditaires ou sporadiques, les lésions neuronales liées au vieillissement, les neuropathies périphériques héréditaires ou lésionnelles, la maladie de Charcot-Marie-Tooth, les neuropathies diabétiques ou induites par les traitement anticancéreux, les épilepsies, les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, les maladies et traumatismes liés à la dégénérescence des motoneurones, les amyotrophies spinales, particulièrement infantiles, la sclérose en plaques, les scléroses latérales amyotrophiques et les traumatismes de la moelle épinière ou des nerfs moteurs périphériques.

11. Utilisation selon l'une quelconque des revendications 8 à 10, **caractérisée en ce que** le médicament neuroprotecteur est destiné au traitement des amyotrophies spinales, particulièrement infantiles, ou des scléroses latérales amyotrophiques.

12. Un composé répondant à la formule I 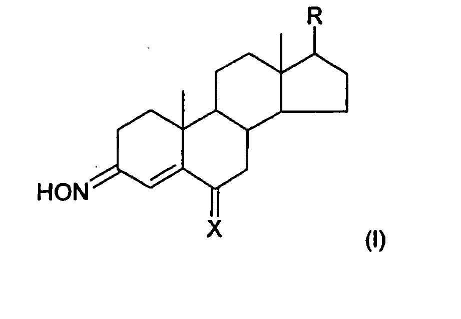 dans laquelle X représente un atome d'oxygène ou un groupement =N-OH et
R représente un groupement choisi parmi 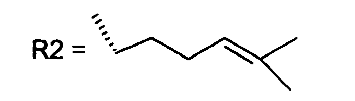 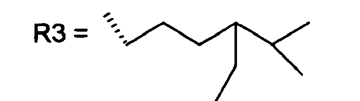 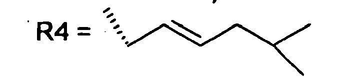 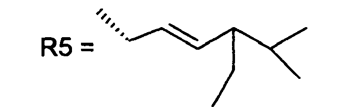 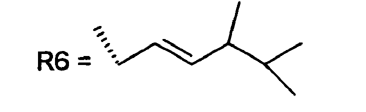 ainsi que ses esters et/ou ses sels d'addition avec les acides minéraux ou organiques.

13. Un composé selon la revendication 12, choisi parmi la 3-oxyimino-cholest-4,24-dièn-6-one, la 24-éthyl-3-oxyimino-cholest-4-èn-6-one, la 3-oxyimino-cholest-4,22-dièn-6-one, la 24-éthyl-3-oxyimino-cholest-4,22-dièn-6-one, la 24-méthyl-3-oxyimino-cholest-4,22-dièn-6-one, la cholest-4,24-dièn-3,6-dioxime, la 24-éthyl-cholest-4-èn-3,6-dioxime, la cholest-4,22-dièn-3,6-dioxime, la 24-éthyl-cholest-4,22-dièn-3,6-dioxime, la 24-méthyl-cholest-4,22-dièn-3,6-dioxime.

14. Procédé de préparation des composés de formule I selon les revendications 12 ou 13, ainsi que de leurs esters, et/ou leurs sels, **caractérisé en ce que** l'on fait réagir un composé de formule II. 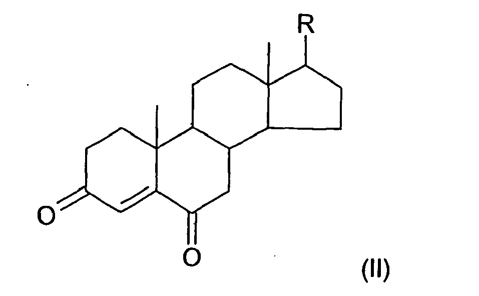 dans laquelle
R représente un groupement choisi parmi 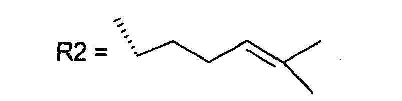 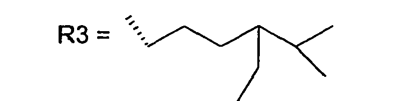 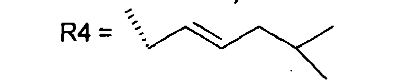 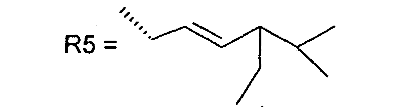 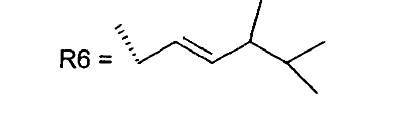 avec un halogénure d'hydroxylamine.

15. Une composition pharmaceutique **caractérisée en ce qu'**elle renferme à titre de principe actif au moins un composé de formule I tel que décrit dans l'une quelconque des revendications 1 à 4, ainsi qu'un excipient pharmaceutiquement acceptable.

## Claims

1. A compound fitting formula I 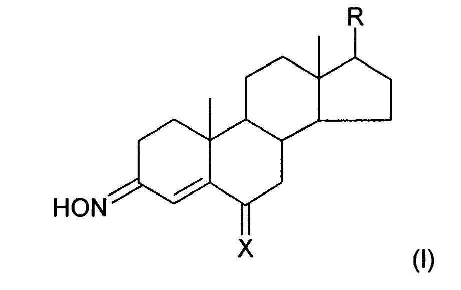 wherein X represents an oxygen atom or a =N-OH group, and
R represents a group selected from 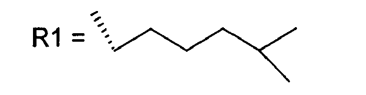 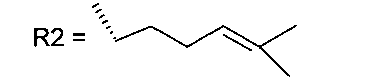 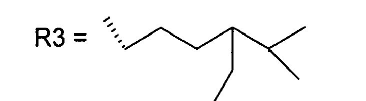 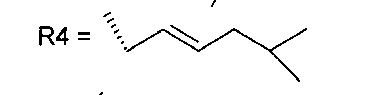 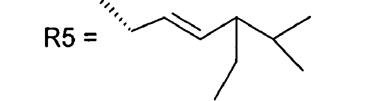 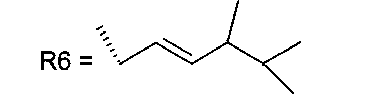 or one of its esters and/or its addition salts with pharmaceutically acceptable acids, for its use as a drug.

2. The compound according to claim 1, **characterized in that** it is selected from 3-oxyimino-cholest-4-en-6-one, 3-oxyimino-cholest-4,24-dien-6-one, 24-ethyl-3-oxyimino-cholest-4-en-6-one, 3-oxyimino-cholest-4,22-dien-6-one, 24-ethyl-3-oxyimino-cholest-4,22-dien-6-one, 24-methyl-3-oxyimino-cholest-4,22-dien-6-one, cholest-4-en-3,6-dioxime, cholest-4,24-dien-3,6-dioxime, 24-ethyl-cholest-4-en-3,6-dioxime, cholest-4,22-dien-3,6-dioxime, 24-ethyl-cholest-4,22-dien-3,6-dioxime, 24-methyl-cholest-4,22-dien-3,6-dioxime, or one of its esters and/or its addition salts with pharmaceutically acceptable acids.

3. The compound according to any of claims 1 or 2, **characterized in that** it is selected from 3-oxyimino-cholest-4-en-6-one, 3-oxyimino-cholest-4,24-dien-6-one, 24-ethyl-3-oxyimino-cholest-4-en-6-one, 3-oxyimino-cholest-4,22-dien-6-one, 24-ethyl-3-oxyimino-cholest-4,22-dien-6-one, 24-methyl-3-oxyimino-cholest-4,22-dien-6-one, or one of its esters and/or its additional salts with pharmaceutically acceptable acids.

4. The compound according to any of claims 1 to 3, **characterized in that** it is selected from 3-oxyimino-cholest-4-en-6-one or 24-ethyl-3-oxyimino-cholest-4-en-6-one, particularly 3-oxyimino-cholest-4-en-6-one, or one of its esters and/or its addition salts with pharmaceutically acceptable acids.

5. The use of a compound of formula I as described in any of claims 1 to 4, for preparing a cytoprotective drug, preferentially a cardioprotective and/or neuroprotective drug.

6. The use of a compound of the above formula I in the preparation of a drug intended for the treatment or prevention of necrosis and/or pathological apoptosis and/or necroptosis (anti-necrotic and/or anti-apoptotic and/or anti-necroptotic drugs).

7. The use of a compound of formula I as described in any of claims 1 to 4, for preparing a drug intended for the treatment of pathologies or traumas related to the degeneration or death of cells, particularly of cardiac cells and/or neurons, whether the latter are natural or accidental.

8. The use of a compound of formula I as described in any of claims 1 to 4, or of one of its esters and/or its addition salts with pharmaceutically acceptable acids, for the preparation of a drug intended for treating diseases such as:
■ diseases of the bones, the joints, the connective tissue and cartilage;
■ muscular diseases such as muscular dystrophy;
■ diseases of the skin;
■ cardiovascular diseases;
■ circulatory diseases;
■ haematological and vascular diseases;
■ lung diseases including obstructive chronic diseases of the lungs;
■ diseases of the gastro-intestinal tract;
■ diseases of the liver;
■ diseases of the pancreas;
■ metabolic diseases;
■ diseases of the kidneys;
■ viral and bacterial infections;
■ severe intoxications;
■ degenerative diseases associated with the Acquired Immune Deficiency Syndrome (AIDS);
■ disorders associated with aging;
■ inflammatory diseases;
■ auto-immune diseases;
■ dental disorders;
■ ophthalmic diseases or disorders;
■ disorders of the audition tracts;
■ diseases associated with mitochondria (mitochondrial pathologies).

9. The use of a compound of formula I as described in any of claims 1 to 4, or of one of its esters and/or of its addition salts with pharmaceutically acceptable acids, for preparing a drug intended for the treatment of neurodegenerative diseases.

10. The use according to claim 8 or 9, **characterized in that** the neuroprotective drug is intended for the treatment of a neurodegenerative disease selected from Huntington's disease, hereditary or sporadic neurodegenerative chronic diseases, neuronal lesions related to aging, hereditary or lesional peripheral neuropathies, Charcot-Marie-Tooth's disease, diabetic neuropathies or those induced by anti-cancer treatments, epilepsies, traumas of the brain, the peripheral nerves or the spinal cord, ischemias of the brain or the spinal cord, hereditary, lesional or aging-related degenerations of sensorial neurons of vision, or degenerations of the optical nerve, hereditary, traumatic or aging-related degenerations of sensorial neurons of hearing, lobar atrophies, and vascular dementias, diseases and traumas related to the degeneration of motoneurons, spinal amyotrophies, in particular infantile spinal amyotrophies, multiple sclerosis; amyotrophic lateral sclerosis, and traumas of the spinal cord and of the peripheral motor nerves.

11. The use according to any of claims 8 to 10, **characterized in that** the neuroprotective drug is intended for the treatment of spinal amyotrophies, in particular infantile spinal amyotrophies, or amyotrophic lateral sclerosis.

12. A compound fitting the formula I 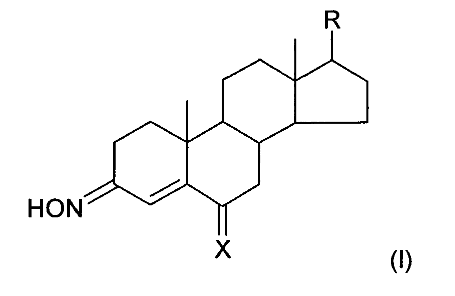 wherein X represents an oxygen atom or a =N-OH group and
R represents a group selected from 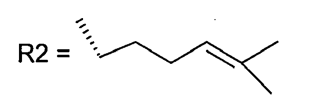 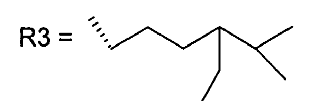 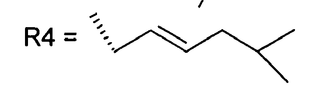 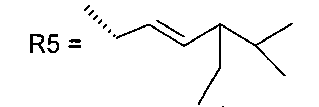 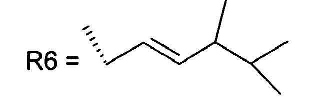 as well as its esters and/or its addition salts with mineral or organic acids.

13. A compound according to claim 12, selected from 3-oxyimino-cholest-4,24-dien-6-one, 24-ethyl-3-oxyimino-cholest-4-en-6-one, 3-oxyimino-cholest-4,22-dien-6-one, 24-ethyl-3-oxyimino-cholest-4,22-dien-6-one, 24-methyl-3-oxyimino-cholest-4,22-dien-6-one, cholest-4,24-dien-3,6-dioxime, 24-ethyl-cholest-4-en-3,6-dioxime, cholest-4,22-dien-3,6-dioxime, 24-ethyl-cholest-4,22-dien-3,6-dioxime, 24-methyl-cholest-4,22-dien-3,6-dioxime.

14. A method for preparing compounds of formula I according to claims 12 or 13, as well as their esters, and/or their salts, **characterized in that** a compound of formula II is reacted 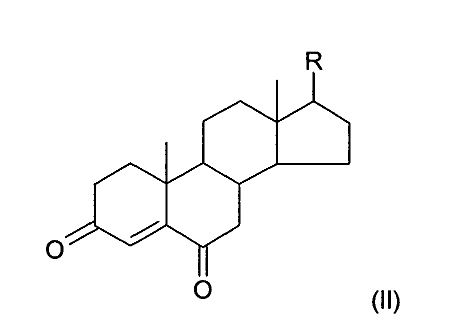 wherein
R represents a group selected from 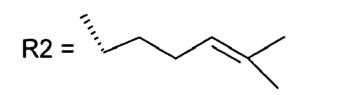 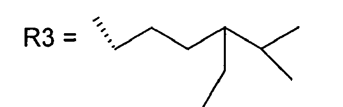 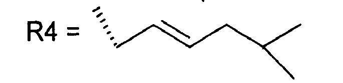 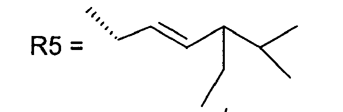 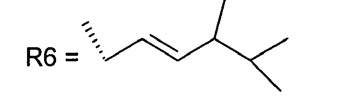 with a hydroxylamine halide.

15. A pharmaceutical composition **characterized in that** it contains as an active ingredient, at least one compound of formula I as described in any of claims 1 to 4, as well as a pharmaceutically acceptable excipient.

## Patentansprüche

1. Verbindung, die der Formel I entspricht: 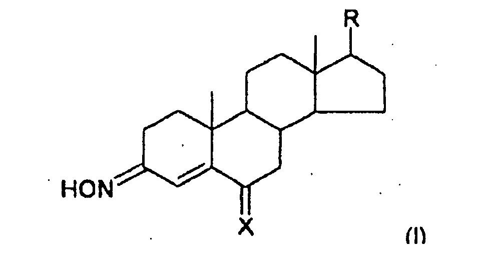 in der X ein Sauerstoffatom oder eine Gruppierung =N-OH darstellt, und
R eine Gruppierung darstellt, die ausgewählt ist aus 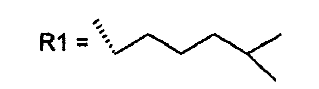 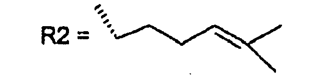 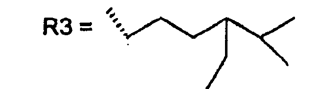 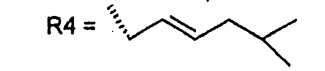 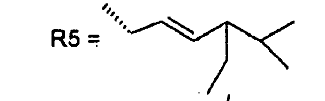 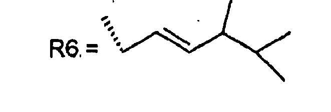 oder einer ihrer Ester und/oder eines ihrer Additionssalze mit pharmazeutisch verträglichen Säuren zur Verwendung als Medikament.

2. Verbindung gemäß Anspruch 1, **dadurch gekennzeichnet, dass** sie ausgewählt ist aus 3-Oxyimino-cholest-4-en-6-on, 3-Oxyimino-cholest-4,24-dien-6-on, 24-Ethyl-3-oxyimino-cholest-4-en-6-on, 3-Oxyimino-cholest-4,22-dien-6-on, 24-Ethyl-3-oxyimino-cholest-4,22-dien-6-on, 24-Methyl-3-oxyimino-cholest-4,22-dien-6-on, Cholest-4-en-3,6-dioxim, Cholest-4,24-dien-3,6-dioxim, 24-Ethyl-cholest-4-en-3,6-dioxim, Cholest-4,22-dien-3,6-dioxim, 24-Ethyl-cholest-4,22-dien-3,6-dioxim, 24-Methyl-cholest-4,22-dien-3,6-dioxim und einem ihrer Ester und/oder einem ihrer Additionssalze mit pharmazeutisch verträglichen Säuren.

3. Verbindung gemäß einem der Ansprüche 1 oder 2, **dadurch gekennzeichnet, dass** sie ausgewählt ist aus 3-Oxyimino-cholest-4-en-6-on, 3-Oxyimino-cholest-4,24-dien-6-on, 24-Ethyl-3-oxyimino-cholest-4-en-6-on, 3-Oxyimino-cholest-4,22-dien-6-on, 24-Ethyl-3-oxyimino-cholest-4,22-dien-6-on, 24-Methyl-3-oxyimino-cholest-4,22-dien-6-on oder einem ihrer Ester und/oder einem ihrer Additionssalze mit pharmazeutisch verträglichen Säuren.

4. Verbindung gemäß einem der Ansprüche 1 bis 3, **dadurch gekennzeichnet, dass** sie ausgewählt ist aus 3-Oxyimino-cholest-4-en-6-on oder 24-Ethyl-3-oxyimino-cholest-4-en-6-on, insbesondere 3-Oxyimino-cholest-4-en-6-on, oder einem ihrer Ester und/oder einem ihrer Additionssalze mit pharmazeutisch verträglichen Säuren.

5. Verwendung einer Verbindung der Formel I, wie sie in einem der Ansprüche 1 bis 4 beschrieben ist, für die Herstellung eines zytoprotektiven, vorzugsweise kardioprotektiven und/oder neuroprotektiven Medikaments.

6. Verwendung einer Verbindung der obigen Formel I bei der Herstellung eines Medikaments, das zur Behandlung oder zur Prävention von Nekrose und/oder pathologischer Apoptose und/oder Nekroptose bestimmt ist (antinekrotische und/oder antiapoptotische und/oder antinekroptotische Medikamente).

7. Verwendung einer Verbindung der Formel I, wie sie in einem der Ansprüche 1 bis 4 beschrieben ist, für die Herstellung eines Medikaments, das zur Behandlung von Pathologien oder Traumata bestimmt ist, die mit der Degeneration oder dem Tod von Zellen, insbesondere Herzzellen und/oder Neuronen verbunden sind, ganz gleich, ob sie natürlich oder zufällig sind.

8. Verwendung einer Verbindung der Formel I, wie sie in einem der Ansprüche 1 bis 4 beschrieben ist, oder einer ihrer Ester und/oder eines ihrer Additionssalze mit pharmazeutisch verträglichen Säuren für die Herstellung eines Medikaments, das zur Behandlung von Erkrankungen wie:
• Erkrankungen der Knochen, der Gelenke, des Bindegewebes und des Knorpels;
• Muskelerkrankungen wie Muskelddystrophie;
• Erkrankungen der Haut;
• kardiovaskuläre Erkrankungen;
• Kreislauferkrankungen;
• hämatologische und vaskuläre Erkrankungen;
• Erkrankungen der Lunge, zum Beispiel chronisch-obstruktive Lungenerkrankungen;
• Erkrankungen des Gastrointestinaltrakts;
• Erkrankungen der Leber;
• Erkrankungen des Pankreas;
• Stoffwechselerkrankungen;
• Nierenerkrankungen;
• virale und bakterielle Infektionen;
• schwere Intoxikationen;
• degenerative Erkrankungen, die mit dem erworbenen Immundefizienzsydrom (AIDS) verbunden sind;
• Störungen, die mit dem Altern verbunden sind;
• inflammatorische Erkrankungen;
• Autoimmunerkrankungen;
• dentale Störungen;
• ophthalmisiche Erkrankungen oder Störungen;
• Störungen der Hörwege;
• Erkrankungen, die mit den Mitochondrien verbunden sind (mitochondriale Pathologien)
bestimmt ist.

9. Verwendung einer Verbindung der Formel I, wie sie in einem der Ansprüche 1 bis 4 beschrieben ist, oder eines ihrer Ester und/oder ihrer Additionssalze mit pharmazeutisch verträglichen Säuren für die Herstellung eines Medikaments, das zur Behandlung neurodegenerativer Erkrankungen bestimmt ist.

10. Verwendung gemäß Anspruch 8 oder 9, **dadurch gekennzeichnet, dass** das neuroprotektive Medikament zur Behandlung einer neurodegenerativen Erkrankung bestimmt ist, die ausgewählt ist aus Huntington'scher Erkrankung, chronischen neurodegenerativen Erkrankungen, die ererbt oder sporadisch sind, neuronalen Läsionen, die mit dem Altern verbunden sind, ererbten oder läsionalen peripheren Neuropathien, der Charcot-Marie-Tooth-Erkrankung, diabetischen Neuropathien oder Neuropathien, die durch Antikrebsbehandlung induziert sind, Epilepsien, Traumata des Gehirns, der peripheren Nerven oder des Rückenmarks, Ischämien des Gehirns oder des Rückenmarks, ererbten, verletzungsbedingten oder mit dem Altern verbundene Degenerationen der sensorischen Neuronen des Sehens oder Degenerationen des Sehnervs, ererbten, traumatischen oder mit dem Altern verbundenen Degenerationen der sensorischen Neuronen des Gehörs, lobären Atrophien und vaskulärer Demenz, Erkrankungen und Traumata, die mit der Degeneration der Motoneuronen verbunden sind, spinalen Amyotrophien, insbesondere bei Kindern, multipler Sklerose, amyotropher Lateralsklerose und Traumata des Rückenmarks oder der peripheren motorischen Nerven.

11. Verwendung gemäß einem der Ansprüche 8 bis 10, **dadurch gekennzeichnet, dass** das neuroprotektive Medikament zur Behandlung der spinalen Amyotrophien, insbesondere bei Kindern, oder der amyotrophen Lateralsklerosen bestimmt ist.

12. Verbindung, die der Formel I entspricht: 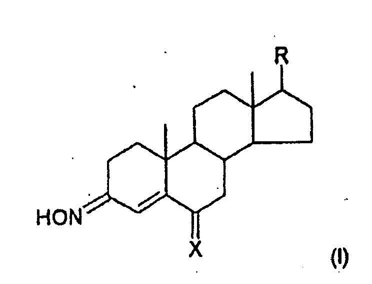 in der X ein Sauerstoffatom oder eine Gruppierung =N-OH darstellt und
R eine Gruppierung darstellt, ausgewählt aus 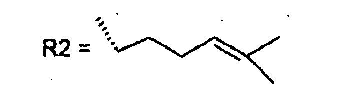 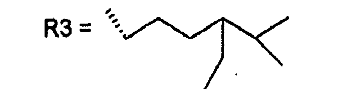 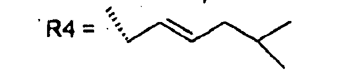 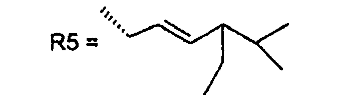 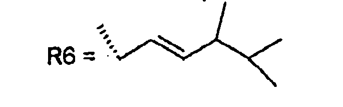 sowie ihre Ester und/oder ihrer Additionssalze mit mineralischen oder organischen Säuren.

13. Verbindung gemäß Anspruch 12, ausgewählt aus 3-Oxyimino-cholest-4,24-dien-6-on, 24-Ethyl-3-oxyimino-cholest-4-en-6-on, 3-Oxyimino-cholest-4,22-dien-6-on, 24-Ethyl-3-oxyimino-cholest-4,22-dien-6-on, 24-Methyl-3-oxyimino-cholest-4,22-dien-6-on, Cholest-4,24-dien-3,6-dioxim, 24-Ethyl-cholest-4-en-3,6-dioxim, Cholest-4,22-dien-3,6-dioxim, 24-Ethyl-cholest-4,22-dien-3,6-dioxim, 24-Methyl-cholest-4,22-dien-3,6-dioxim.

14. Verfahren zur Herstellung der Verbindungen der Formel I gemäß Anspruch 12 oder 13 sowie ihrer Ester und/oder ihrer Salze, **dadurch gekennzeichnet, dass** man eine Verbindung der Formel II 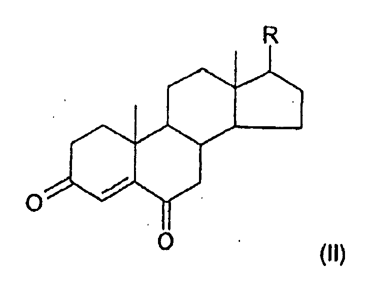 in der R eine Gruppierung darstellt, ausgewählt aus 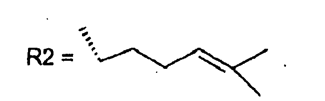 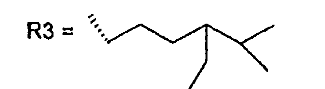 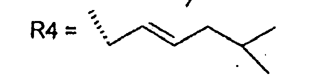 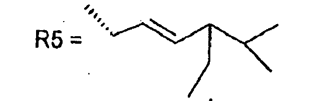 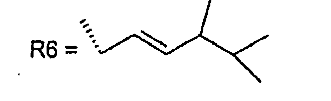 mit einem Hydroxylaminhalogenid reagieren lässt.

15. Pharmazeutische Zusammensetzung, **dadurch gekennzeichnet, dass** sie als Wirkstoff wenigstens eine Verbindung der Formel I, wie sie in einem der Ansprüche 1 bis 4 beschrieben ist, sowie ein pharmazeutisch verträgliches Exzipiens umfasst.
